# EUROPÄISCHE PATENTSCHRIFT

(11) **EP 4 446 014 B1**
(45) Veröffentlichungstag und Bekanntmachung des Hinweises auf die Patenterteilung: **22.07.2026**
(21) Anmeldenummer: 23168076.0
(22) Anmeldetag: 14.04.2023
(51) Int. Cl.: B05B 11/00

(54) **FLÜSSIGKEITSSPENDER**
FLUID DISPENSER
DISTRIBUTEUR DE LIQUIDE

(43) Veröffentlichungstag der Anmeldung: 16.10.2024
(73) Patentinhaber: Aptar Radolfzell GmbH, 78315 Radolfzell (DE)
(72) Erfinder: Welsch, Leonard, 76131 Karlsruhe (DE)
(74) Vertreter: Witte, Weller und Partner Patentanwälte mbB Stuttgart

(56) Entgegenhaltungen:
- WO-A1-01/03851
- JP-A- 2004 223 515

## Beschreibung

### ANWENDUNGSGEBIET UND STAND DER TECHNIK

Die Erfindung betrifft einen üblicherweise mobilen Flüssigkeitsspender, mittels dessen Flüssigkeiten in vorzugsweise zerstäubter Form ausgetragen werden. Die Bauweise eines erfindungsgemäßen Flüssigkeitsspenders eignet sich für verschiedene medizinische Anwendungen, aber auch für nicht-medizinische Anwendungen wie den Austrag von nikotinhaltigen Flüssigkeiten.

Bei Flüssigkeitsspendern, die von ihrem Benutzer häufig verwendet werden, beispielsweise Flüssigkeitsspender zum Austrag von nikotinhaltigen oder cannabishaltigen Produkten oder von Schmerzmitteln, ist gewünscht, dass diese möglichst bequem bedienbar sind, also möglichst über einen einfachen und eingeübten Handgriff genutzt werden können. Es ist aber auch gewünscht, dass die Flüssigkeitsspender hierfür zunächst in einen Nutzzustand überführt werden, in dem der Austrag dann stattfinden kann. Dieses Erfordernis der initialen Überführung in einen Nutzzustand soll verhindern, dass der Flüssigkeitsspender ungeplant Flüssigkeit abgibt oder dass er durch Kleinkinder zu einfach zu bedienen ist. Siehe Dokument JP2004223515.

Dokument US 2004/0155067 A1 beschreibt einen Flüssigkeitsspender mit einem Flüssigkeitsspeicher, einer Fördereinrichtung und einem Flüssigkeitsapplikator. Der Applikator ist in einer Schutzstellung durch ein schwenkbares Abdeckelement geschützt. Dieses Abdeckelement übernimmt zugleich die Funktion eines Betätigungselements zur Auslösung der Fördereinrichtung.

Dokument WO 01/03851 A1 offenbart einen Flüssigkeitsspender mit einem Flüssigkeitsapplikator und einer Fördereinrichtung, bei dem eine schwenkbare Schutzkappe vorgesehen ist. Die Schutzkappe deckt den Applikator in einer Schutzstellung ab und dient in einer geöffneten Stellung zugleich als Betätigungselement zur Auslösung der Fördereinrichtung.

### AUFGABE UND LÖSUNG

Aufgabe der Erfindung ist es, einen Flüssigkeitsspender zur Verfügung zu stellen, der eine einfache Art der Bedienung ermöglicht, der aber gleichzeitig derart ausgestaltet werden kann, dass er wirksam versehentliche Betätigungen und/oder Betätigung durch Kleinkinder verhindert.

Zur Lösung dieser Aufgabe wird ein Flüssigkeitsspender nach Anspruch 1 vorgeschlagen.

Vorzugsweise ist die Fördereinrichtung eine Pumpeinrichtung, die Flüssigkeit aus einem drucklosen Flüssigkeitsspeicher ansaugt und austrägt. Alternativ kann die Fördereinrichtungjedoch auch durch ein schlichtes Auslassventil gebildet sein, welches zuvor unter Druck gespeicherte Flüssigkeit aus einem als Druckspeicher ausgebildeten Flüssigkeitsspeicher entweichen lässt, sobald das Ventil geöffnet ist.

Der Flüssigkeitsspender dient insbesondere dem Austrag von Flüssigkeit in zerstäubter Form, wobei dies vorzugsweise durch eine Sprühgeometrie mit einer der Austragöffnung vorgeschalteten Wirbelkammer oder durch eine Düseneinheit mit einer Vielzahl feiner Düsenöffnungen erreicht wird. Es sind medizinische und nicht-medizinische Anwendungsfelder für einen Flüssigkeitsspender der hier beschriebenen Art vorgesehen. Insbesondere für medizinische Anwendungen ist eine Gestaltung vorgesehen, bei der der Flüssigkeitsapplikator in Art eines Nasalapplikators länglicher und sich verjüngender Form ausgebildet ist. Insbesondere für nicht-medizinische Anwendungen, nämlich für den Konsum von nikotin- oder cannabishaltigen Flüssigkeiten, ist die Gestaltung mit einem zylindrischen Oralapplikator vorgesehen.

Der Flüssigkeitsspender ist als mobiler Flüssigkeitsspender ausgebildet, den ein Benutzer üblicherweise mit sich führt. Ein Volumen des Flüssigkeitsspeichers ist daher gering. Es liegt vorzugsweise bei weniger als 50 ml, insbesondere vorzugsweise bei weniger als 10 ml. Seine Länge beträgt vorzugsweise weniger als 150 mm, insbesondere weniger als 120 mm.

Der Flüssigkeitsspender als Ganzes weist vorzugsweise eine längliche Form auf. Er ist entlang einer Längsachse vorzugsweise Faktor 2 größer als in Querrichtung hierzu. Die Längsseiten des Flüssigkeitsspenders dienen insbesondere dem Umgreifen mit einer Hand. An einer Stirnseite des Flüssigkeitsspenders ist vorzugsweise der Flüssigkeitsapplikator vorgesehen.

Um im mitgeführten Zustand den Flüssigkeitsapplikator des Flüssigkeitsspender zu schützen, verfügt der Flüssigkeitsspender über eine Applikatoreinheit, an der der Flüssigkeitsapplikator vorgesehen ist, und über eine Schutzeinheit mit einer Schutzhülle zum Schutz der Applikatoreinheit. Die Schutzeinheit ist unverlierbarer Teil des Flüssigkeitsspenders. Er bleibt bei normaler Nutzung des Flüssigkeitsspenders stets mit der Applikatoreinheit verbunden.

Die Schutzeinheit und die Applikatoreinheit sind zwischen einer Schutzstellung und einer Nutzstellung gegeneinander verlagerbar, vorzugweise linear und entlang einer Hauptachse des Flüssigkeitsspenders. In der Schutzstellung umgibt die Schutzeinheit den Flüssigkeitsapplikator zumindest teilweise und schützt ihn hierdurch. In der durch manuell bewirkte Relativverlagerung erzielbaren Nutzstellung befindet sind der Flüssigkeitsapplikator relativ zur Schutzeinheit in einer exponierten Position und ist zumindest partiell aus der Schutzhülle herausverlagert. In dieser Position ist nun der Flüssigkeitsaustrag möglich. Die Distanz, um die die Applikatoreinheit und die Schutzeinheit gegeneinander verlagerbar sind, beträgt vorzugsweise mindestens 5 mm, insbesondere vorzugsweise mindestens 10 mm oder gar mindestens 15 mm.

Sowohl für die Relativverlagerung der Schutzeinheit und der Applikatoreinheit gegeneinander als auch zur Erzielung des Flüssigkeitsaustrags selbst ist eine gegenüber der Applikatoreinheit und gegenüber der Schutzeinheit bewegliche Bedienhandhabe vorgesehen. Diese kann linear beweglich sein, ist aber vorzugweise als schwenkbeweglicher Bedienhebel schwenkbeweglich gegenüber der Schutzeinheit und der Applikatoreinheit angebracht. Die Verlagerung der Bedienhandhabe erfolgt zumindest phasenweise in einer zur Relativverlagerungsrichtung von Schutzeinheit und Applikatoreinheit anwinkelten Richtung, vorzugsweise um einen Winkel von mindestens 75° angewinkelt.Mittels der Bedienhandhabe kann die Schutzeinheit gegenüber der Applikatoreinheit aus der Schutzstellung in die Nutzstellung verlagert werden. Die Bedienhandhabe ist demnach so angebracht und ausgelegt, dass sie die Einkopplung der zur Relativverlagerung aus der Schutzstellung in die Nutzstellung erforderliche Kraft auf die Schutzeinheit und die Applikatoreinheit gestattet. Die Betätigungshandhabe ist vorzugsweise an einer Längsseite des Flüssigkeitsspenders vorgesehen. Die Nutzung erfolgt bestimmungsgemäß derart, dass der Nutzer die Applikatoreinheit umgreift und mit einem Finger oder insbesondere dem Daumen die Betätigungshandhabe kraftbeaufschlagt, um die Schutzeinheit in die Nutzstellung zu überführen, und direkt im Anschluss die Betätigungshandhabe nochmals kraftbeaufschlagt, um den Flüssigkeitsaustrag zu bewirken, indem mittels der Betätigungshandhabe die Fördereinrichtung betätigt und Flüssigkeit aus dem Flüssigkeitsspeicher zum Flüssigkeitsapplikator gefördert wird.

Die beschriebene Bauweise des Flüssigkeitsspenders gestattet eine sehr bequeme Bedienung, ist aber gleichzeitig sehr gut geeignet, um Schutz gegen versehentliche Überführung in die Nutzstellung oder gar einen unbeabsichtigten Flüssigkeitsaustrag zu bieten. Die Verwendbarkeit der Bedienhandhabe für die Herstellung des Nutzzustandes und für den Flüssigkeitsaustrag gestattet es, mit einer Bewegung beides zu erzielen.

Obwohl eine bequeme Bedienung bei dem erfindungsgemäßen Flüssigkeitsspender angestrebt wird, verfügt der Flüssigkeitsspender vorzugsweise über eine Blockiereinrichtung, die um Zuge der Benutzung gelöst werden muss, und die eine Verlagerung der Schutzeinheit und der Applikatoreinheit aus der Schutzstellung in die Nutzstellung zu gestatten. Die Blockiereinrichtung ist vorzugsweise ebenfalls mittels der Bedienhandhabe lösbar, so dass anschließend die Verlagerung der Schutzeinheit und der Applikatoreinheit in die Nutzstellung bewirkt werden kann.

Zweck der Blockiereinrichtung ist es, eine ungewollte Überführung des Flüssigkeitsspenders in die Nutzstellung zu verhindern. Dies betrifft einerseits eine ungewollte Überführung, die durch unabsichtliche Kraftbeaufschlagung in einer Tasche oder dergleichen verursacht ist. Je nach Ausgestaltung kann eine Blockiereinrichtung jedoch auch als Kindersicherung dienen, die zumindest kleine Kinder wirksam von der Herstellung des Nutzzustandes abhält.

Die Blockiereinrichtung ist vorzugsweise derart ausgebildet, dass eine Verlagerung der Schutzeinheit gegenüber der Applikatoreinheit erst in relevantem Maße beginnen kann, nachdem zuvor die Bedienhandhabe betätigt worden ist. Die Bedienhandhabe muss also zur Verwendung des Flüssigkeitsspeichers initial verlagert werden, um hierdurch die Verschiebung der Schutzeinheit zu ermöglichen. Ist die Nutzstellung erreicht, erfolgt eine weitere Kraftbeaufschlagung der Bedienhandhabe zum Zwecke des Flüssigkeitsaustrags. Vorzugsweise sind einander entgegengesetzte Bewegungen der Bedienhandhabe zur Lösung der Blockiereinrichtung und zur Erzielen des Flüssigkeitsaustrags vorgesehen.

Die Bedienhandhabe ist vorzugsweise derart mit der Blockiereinrichtung gekoppelt, dass durch eine Verlagerung der Bedienhandhabe nach außen und insbesondere in einer vom Flüssigkeitsapplikator wegweisenden Richtung die Blockiereinrichtung gelöst werden kann. Dies führt dazu, dass die Gesamtbewegung zum Lösen der Blockierung und zum Herstellen des Nutzzustandes eine erste Teilbewegung der Bedienhandhabe in einer ersten Richtung zur Lösung der Blockierung und eine zweite Teilbewegung der Bedienhandhabe in einer abweichenden zweiten Richtung zur Verlagerung der Schutzeinheit umfasst. Für kleine Kinder ist die Sequenz schwer durchzuführen, insbesondere wenn die erste Teilbewegung gegen eine Federkraft erfolgt und somit ein Umgreifen des Kindes für die zweite Teilbewegung erschwert wird, da die Bedienhandhabe beim Loslassen in ihre blockierende Ausgangsstellung zurückkehrt.

Die Bedienhandhabe dient wie beschrieben auch der Kraftbeaufschlagung der Applikatoreinheit und der Schutzeinheit gegeneinander. Wenngleich die Bedienhandhabe gegenüber beiden Einheiten beweglich ist, ist sie jedoch vorzugsweise gemeinsam mit der Schutzeinheit gegenüber der Applikatoreinheit verlagerbar. Die Bedienhandhabe ist also bezüglich der Verlagerungsrichtung derart mit der Schutzeinheit gekoppelt, dass eine Kraftübertragung in Verlagerungsrichtung möglich ist.

Insbesondere kann zu diesem Zweck eine Kulissenführung vorgesehen sein, mittels derer die Bedienhandhabe beweglich an der Schutzeinheit geführt ist. Die Kulissenführung ermöglicht gleichzeitig eine Bewegbarkeit der Bedienhandhabe gegenüber der Schutzeinheit und die gemeinsame Bewegbarkeit mit der Schutzeinheit gegenüber der Applikatoreinheit. Die Kulissenführung erstreckt sich vorzugsweise orthogonal zur Verlagerungsrichtung der Schutzeinheit und der Applikatoreinheit, schließt mit dieser mindestens jedoch einen Winkel von 70° ein.

Wie bereits erläutert, ist es bevorzugt, wenn derschwenkbewegliche Bedienhebel um eine Schwenkachse schwenkbar an der Applikatoreinheit gelagert ist, die an einem vom Flüssigkeitsapplikator wegweisenden Ende des Bedienhebels vorgesehen ist. Dies bedeutet, dass eine Schwenkbewegung des Hebels nach Erreichen der Nutzlage um eine zur Applikatoreinheit im Wesentlichen ortsfeste Schwenkachse erfolgt. Das gegenüberliegende verschwenkende Ende des Bedienhebels weist zum Ende des Flüssigkeitsspenders, an dem der Flüssigkeitsapplikator und die Schutzhülle vorgesehen sind.

Der schwenkbewegliche Bedienhebel wird vorzugsweise ausgehend von einer Ruhelage nach außen und/oder von dem Nasalapplikator weg verschwenkt werden, um die genannte Blockiereinrichtung zu lösen. Um den Bedienhebel nach außen verschwenken zu können, ist vorzugsweise eine Greiferhebung an einem distalen Ende des Bedienhebels vorgesehen, die sich über eine Stirnseite des Flüssigkeitsspenders erstreckt und einfach erfassbar ist.

Der schwenkbewegliche Bedienhebel wird vorzugsweise ausgehend von einer ausgeschwenkten Lage nach innen und/oder in Richtung des Nasalapplikators verschwenkt werden, um die Fördereinrichtung zu betätigen. Insbesondere kann der Bedienhebel die Bewegung nach innen, insbesondere über ein Umlenkungsgetriebe, auf ein Auslassventil oder einen Pumpenkolben der Fördereinrichtung wirken.

Wenn der Bedienhebel bezogen auf die Verlagerungsrichtung der Applikatoreinheit und der Schutzeinheit an der Schutzeinheit befestigt ist, wie oben beschrieben, so wird seine Schwenkachse während der Verlagerung relativ zur Applikatoreinheit verlagert. Die Schwenkachse ist zu diesem Zweck vorzugsweise geführt beweglich an der Applikatoreinheit befestigt. Insbesondere kann eine Kulissenführung vorgesehen sein, mittels derer die Verschiebung der Schwenkachse gegenüber der Applikatoreinheit gesteuert ist. Die Kulissenführung ist vorzugsweise an einem Gehäuse der Applikatoreinheit vorgesehen.

Eine besonders vorteilhafte Gestaltung sieht vor, dass die Führung eine zumindest abschnittsweise von der Verlagerungsrichtung der Schutzeinheit gegenüber der Applikatoreinheit abweichende Erstreckungsrichtung aufweist. Dies führt dazu, dass die Schwenkachse des Bedienhebels bei der Überführung der Schutzeinheit aus der Schutzstellung in die Nutzstellung abweichend von der Verlagerungsrichtung verlagert wird. Hierdurch kann erzielt werden, dass das Ende des Bedienhebels in das Gehäuse der Applikatoreinheit eintaucht. Es kann hierdurch der Bedarf vermieden werden, an der Applikatoreinheit eine in der Schutzstellung freie Öffnung zu schaffen, um den Bedienhebel nach Überführung in die Nutzstellung aufzunehmen. Da es bevorzugt ist, dass der Schutzabschnitt, der Applikatorabschnitt und der Bedienhebel gemeinsam eine bei Nichtgebrauch in der Schutzstellung geschlossene Körperform bilden, die das Eindringen von Schmutz oder das Austreten von Flüssigkeit vermeidet, ist die Vermeidung einer außenliegenden Öffnung für den Bedienhebel hilfreich.

Die bereits erwähnte Blockiereinrichtung kann auf verschiedene Weisen realisiert sein, wobei bevorzugt ist, dass die beiden nachfolgend beschriebenen unabhängigen Teileinrichtungen gemeinsam realisiert sind.

Die Blockiereinrichtung kann insbesondere eine Schutzblende umfassen, die mittels der Bedienhandhabe aus einer Stellung in fluchtender Anordnung zum Flüssigkeitsapplikator und in eine demgegenüber versetzten Stellung verlagerbar ist. In der fluchtenden Anordnung verhindert die Schutzblende die Überführung in den Nutzzustand, da der Flüssigkeitsapplikator nicht ausgefahren werden kann, sondern gegen die Schutzblende stoßen würde.

Die genannte Durchbrechung kann mit der genannten Schutzblende verschließbar sein. Die Schutzblende kann hierfür insbesondere in einer Führung der Schutzeinheit zwischen der Öffnungsstellung und der Schließstellung verlagerbar sein.

Insbesondere weist die Schutzeinheit eine mit einer Durchbrechung versehene Stirnwandung auf. Durch diese Durchbrechung ist der Flüssigkeitsapplikator in der Nutzstellung herausgeschoben. In der Schutzstellung dagegen ist der Flüssigkeitsapplikator eingezogen und vollständig innerhalb eines durch die Stirnwandung begrenzten Innenraums. Diese Isolierung des Flüssigkeitsapplikators im genannten Innenraum in der Schutzstellung ist insbesondere bei Flüssigkeitsspendern vorteilhaft, die für die Zerstäubung der Flüssigkeit eine Düseneinheit mit einer Vielzahl feiner Düsenöffnungen aufweisen. Hier ist die Gefahr durch Verschmutzung besonders relevant. Die Isolierung verhindert oder vermindert eine Verschmutzung.

Die bevorzugte Ausgestaltung der Düseneinheit sieht vor, dass diese vorzugsweise über mindestens 10 Düsenöffnungen verfügt mit einem minimalen lichten Querschnitt von jeweils höchstens 0,02 mm². Vorzugsweise sind mindestens 25 Düsenöffnungen vorgesehen.

Die Schutzblende kann mittels der Bedienhandhabe in die öffnende Stellung verlagert werden, so dass dann das Ausfahren des Flüssigkeitsapplikators möglich ist. Dabei ist die Schutzblende vorzugsweise mit der Bedienhandhabe nur unidirektional gekoppelt, so dass die Schutzblende in die Öffnungsstellung verlagert werden kann, bei einer Bewegung der Bedienhandhabe in entgegengesetzter Richtung jedoch in der Öffnungsstellung verbleiben kann. Hierdurch wird vermieden, dass bei einer Betätigung der Bedienhandhabe zum Zwecke des Flüssigkeitsaustrags die Schutzblende gegen den Flüssigkeitsapplikator stößt und dadurch eine fortgesetzte Verlagerung der Bedienhandhabe unterbindet.

Der Schutzblende ist vorzugsweise ein Federmittel zugeordnet, welches die Schutzblende stets in Richtung der Schließstellung kraftbeaufschlagt. Sobald der Flüssigkeitsspender aus der Nutzstellung in die Schutzstellung gebracht wird, drückt dieses Federmittel die Schutzblende in die Schließstellung und stellt somit die Blockierung wieder her sowie verschließt die ggf. vorgesehen Durchbrechung in der Stirnwandung.

Eine andere Form einer Blockiereinrichtung kann im Bereich der Bedienhandhabe selbst vorgesehen sein. Die Bedienhandhabe und die Applikatoreinheit können Anschlagsflächen aufweisen, die in der Schutzstellung und bei eingedrückter Bedienhandhabe bzw. insbesondere eingeschwenktem Bedienhebel das Verschieben der Schutzeinheit gegenüber der Applikatoreinheit unterbinden. Wenn der Bedienhebel ausgeschwenkt wird oder die Bedienhandhabe herausgezogen ist, gelangen die Anschlagsflächen außer Eingriff und gestatten die Verlagerung der Schutzeinheit.

Vorzugsweise ist die Anschlagsfläche der Bedienhandhabe in einer ersten Vertiefung der Bedienhandhabe vorgesehen. Die Anschlagsfläche der Applikatoreinheit muss zunächst diese Vertiefung verlassen, bevor die Überführung in die Nutzstellung beginnen kann. Vorzugsweise ist eine zweite Vertiefung an der Bedienhandhabe vorgesehen, in die die Anschlagsfläche der Applikatoreinheit einrückt, wenn mittels der Bedienhandhabe die Fördereinrichtung betätigt wird. Diese zweite Aussparung dient also dem Zweck, eine betätigungsbehindernde Kollision bei der Austragsbetätigung in der Nutzstellung zu verhindern.

Insbesondere kann ein nachfolgend beschriebenes Zwischenelement die Funktion der applikatoreinheitsseitigen Anschlagsfläche übernehmen.

Ein solches Zwischenelement ist vorzugsweise zunächst dafür vorgesehen, in der Nutzstellung des Flüssigkeitsspenders die Betätigung der Bedienhandhabe auf die Fördereinrichtung zu übertragen. Insbesondere kann das Zwischenelement als schwenkbeweglicher Zwischenhebel ausgestaltet sein, mittels dessen die Verlagerung der Bedienhandhabe in einer Betätigungsrichtung in eine Kraftbeaufschlagung der Fördereinrichtung in einer von der Betätigungsrichtung abweichenden Richtung überführt wird. Das Zwischenelement kann insbesondere eine quer zur Kolbenbewegungsrichtung erfolgende Betätigungsbewegung umlenken, insbesondere um 90°.

Dieses Zwischenelement kann einen Teil der Blockiereinrichtung bilden. Hierfür kann die applikatoreinheitsseitige Anschlagsfläche an diesem Zwischenelement vorgesehen sein, insbesondere an einem Achsabschnitt, mittels dessen das Zwischenelement schwenkbar in ein Gehäuse der Applikatoreinheit eingesetzt ist.

Ein erfindungsgemäßer Flüssigkeitsspender kann als Einweg-Spender ausbildet sein. Bevorzugt ist jedoch die Wiederverwendung wesentlicher Teile des Flüssigkeitsspenders. Insbesondere vorzugsweise werden insbesondere ein Gehäuse der Applikatoreinheit, die Schutzeinheit sowie die Bedienhandhabe wiederverwendet. Diese zur Wiederverwendung vorgesehenen Teile bilden eine Haupteinheit.

Insbesondere kann vorgesehen sein, dass der Flüssigkeitsspender über eine wechselbare Flüssigkeitskartusche verfügt. Diese kann im einfachsten Falle durch einen Speicherkörper gebildet werden, der an eine in der Haupteinheit verbleibende Fördereinrichtung angekoppelt wird.

Vorzugsweise jedoch umfasst die wechselbare Flüssigkeitskartusche neben einem den Flüssigkeitsspeicher definierenden Speicherkörper einen Austragstutzen und eine Fördereinrichtung. All diese Teile werden demnach getauscht, wenn ein Wechsel der Flüssigkeitskartusche stattfindet. Die Fördereinrichtung, die insbesondere als Pumpeinrichtung oder als Ventileinrichtung ausgebildet ist, kann in einem solchen Fall derart ausgestaltet sein, dass sie durch Relativbewegung des Austragstutzens gegenüber dem Speicherkörper betätigt werden kann.

Dies kann insbesondere genutzt werden, indem der Austragstutzen im eingesetzten Zustand der Flüssigkeitskartusche ortsfest an der Applikatoreinheit verbleibt, während mittels der Bedienhandhabe der Speicherkörper verlagerbar wird, so dass er gegen den Austragstutzen drückt und dadurch die Fördereinrichtung betätigt, also einen Pumpvorgang bewirkt oder ein Auslassventil öffnet.

Der Flüssigkeitsapplikator selbst ist Teil der Applikatoreinheit, kann aber als lösbares Teil ausgebildet sein. Vorzugsweise ist der Flüssigkeitsapplikator im eingesetzten Zustand in eine Aussparung der Applikatoreinheit eingesetzt, aus der er werkzeuglos entfernbar ist. Vorzugsweise sitzt der Flüssigkeitsapplikator lose in der Aussparung, so dass er nach oder mit Entfernen der Flüssigkeitskartusche aus der Aussparung herausgleiten kann. Damit ist der Flüssigkeitsapplikator einfach austauschbar. Er kann darüber hinaus auch selbst Teil der Flüssigkeitskartusche sein, insbesondere indem er klemmend auf den Austragstutzen der Flüssigkeitskartusche aufgesetzt wird.

Der Flüssigkeitsspeicher bzw. die Flüssigkeitskartusche sind im Betrieb vorzugsweise in einem Innenraum der Applikatoreinheit angeordnet. Der Austausch ist möglich, indem eine Verschlussklappe der Applikatoreinheit geöffnet wird. Diese Klappe kann insbesondere an einer dem Flüssigkeitsapplikator abgewandten Seite des Gehäuses oder an einer Längsseite vorgesehen sein.

Der erfindungsgemäße Flüssigkeitsspender kann in der schon beschriebenen Weise partiell zur Wiederverwendung vorgesehen sein. Es wird daher als zweckmäßig angesehen, Sets zusammenzustellen, die neben einem Flüssigkeitsspender mitsamt Flüssigkeitskartusche mindestens eine weitere Flüssigkeitskartusche umfassen. Die Erfindung betrifft aufgrund der möglichen Wiederverwendbarkeit auch einen Flüssigkeitsspender der beschriebenen Art ohne die Flüssigkeitskartusche, umfassend mindestens die Applikatoreinheit ohne Flüssigkeitsspeicher, die Schutzeinheit und die Bedienhandhabe. Aufgrund der Wiederverwendbarkeit wird es als vorteilhaft angesehen, dass die durch diese Komponenten gebildeten Haupteinheit robust und hochwertig ausgebildet ist. Dies bedeutet, dass vorzugsweise mindestens einige der Außenflächen metallisch oder anderweitig aus anderen Materialien als Kunststoff bestehen.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt ein Ausführungsbeispiel eines erfindungsgemäßen Flüssigkeitsspender in einer Gesamtdarstellung.
Fig. 2 zeigt wesentliche Baugruppen des Flüssigkeitsspenders in teilweise isolierter Form.
Fig. 3 bis 5 zeigen die Überführung des Flüssigkeitsspenders aus einem Schutzzustand in einen Nutzzustand.
Fig. 6 verdeutlicht die Einbaulage einer Feder als Teil einer Blockiereinrichtung des Flüssigkeitsspenders.

### DETAILLIERTE BESCHREIBUNG DER AUSFÜHRUNGSBEISPIELE

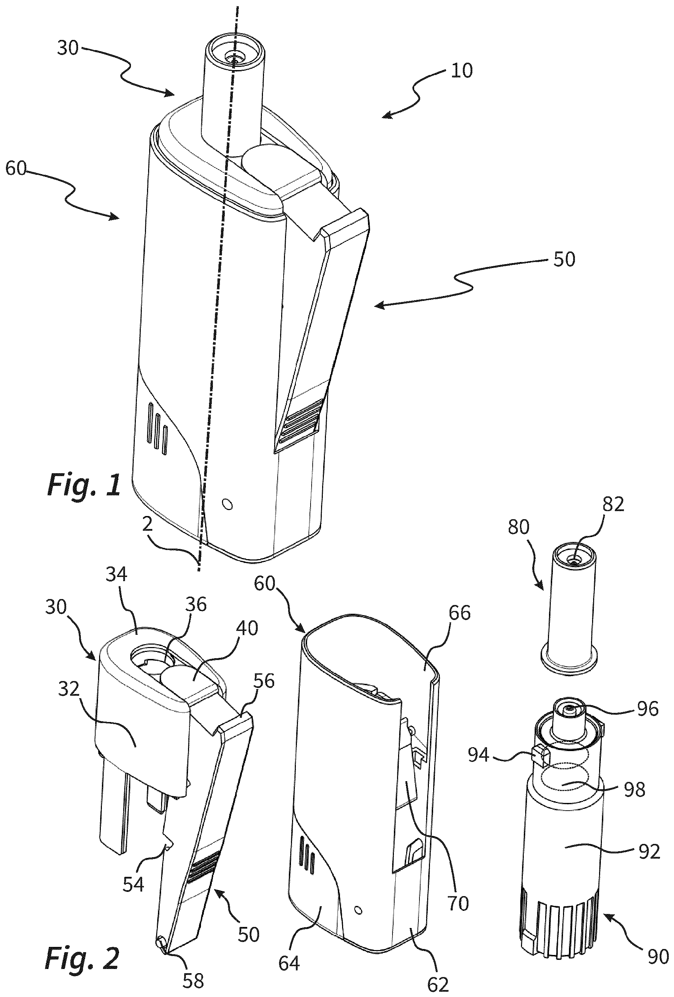

Fig. 1 zeigt einen erfindungsgemäßen Flüssigkeitsspender 10 in perspektivischer Gesamtdarstellung. Der Flüssigkeitsspender 10 kann, wie im Weiteren noch im Detail erläutert wird, in einen Schutzzustand gebracht werden, in dem der Flüssigkeitsspender und insbesondere seine Düseneinheit 82 geschützt ist. Er kann hiervon ausgehend in einen Nutzzustand gebracht werden, in dem er verwendet werden und Flüssigkeit ausgetragen werden kann. Die Fig. 1 zeigt den Nutzzustand.

Fig. 2 zeigt Baugruppen des Flüssigkeitsspenders 10 in teilweise zerlegter Form.

Der Flüssigkeitsspender 10 verfügt über drei primär erfindungswesentliche Baugruppen, die grundsätzlich gegeneinander beweglich sind. Diese Baugruppen sind die einer Applikatoreinheit 60, einer Schutzeinheit 30 und einer Bedienhandhabe 50, vorliegend ausgestaltet in Art eines Bedienhebels 50. Als weitere Baugruppe ist eine austauschbare Flüssigkeitskartusche 90 vorgesehen. Vorliegend ebenfalls als separate Baugruppe dargestellt ist ein Flüssigkeitsapplikator 80, der jedoch im Betrieb einen Teil der Applikatoreinheit 60 bildet und mit deren Gehäuse 62 zusammen verlagerbar ist. Der Flüssigkeitsapplikator 80 kann jedoch als einfach austauschbare Einheit ausgebildet sein.

Wie sich aus Fig. 1 ersehen lässt, ist der Flüssigkeitsspender 10 als insgesamt länglicher Spender mit schlanker Form ausgestaltet, dessen Haupterstreckungsrichtung durch die Längsrichtung 2 gebildet wird. An einer oberen Stirnseite ist der bereits genannte Flüssigkeitsapplikator 80 vorgesehen. An einer Längsseite ist der Bedienhandhabe 50 in Form eines Bedienhebels 50 vorgesehen.

Der Flüssigkeitsspender 10 verfügt neben der Applikatoreinheit 60, die in Fig. 2 in der Mitte dargestellt ist, über die genannte Schutzeinheit 30, die in Fig. 2 links gemeinsam mit der Bedienhandhabe 50 dargestellt ist. Die Schutzeinheit 30 weist eine Schutzhülle 32 sowie eine Stirnwandung 34 auf. In der Nutzstellung der Fig. 1 taucht die Schutzeinheit 30 in die Applikatoreinheit 60 ein und gibt hierdurch den Flüssigkeitsapplikator 80 zur Verwendung frei, der durch eine Durchbrechung 36 in der Stirnwandung 34 der Schutzeinheit 30 nach außen gelangt. Wie anhand der Fig. 2 zu ersehen ist, ist an der Schutzeinheit 30 weiterhin eine Schutzblende 40 vorgesehen, die diese Durchbrechung 36 im Schutzzustand verschließen kann.

In Fig. 2 gemeinsam mit der Schutzeinheit dargestellt ist die Bedienhandhabe 50 in Form eines Bedienhebels 50. Der Bedienhebel 50 ist gegenüber der Schutzeinheit 30 begrenzt beweglich. Bezogen auf die Längsrichtung 2 ist der Bedienhebel 50 jedoch mit der Schutzeinheit 30 gekoppelt, da sein oberes Ende in einer Kulisse 38 unterhalb der Stirnwandung 34 der Schutzeinheit 30 geführt ist. Quer zur Längsrichtung 2 ist der Bedienhebel 50 jedoch gegenüber der Schutzeinheit beweglich, um die im Weiteren noch erläuterten Bewegungsabläufe zu ermöglichen.

Die Applikatoreinheit 60 weist ein Gehäuse 62 auf, dessen Innenraum zur Aufnahme der Flüssigkeitskartusche 90 vorgesehen ist. Zu diesem Zweck ist an der dem Flüssigkeitsapplikator 80 abgewandten Seite des Flüssigkeitsspenders 10 eine abnehmbare Klappe 64 vorgesehen. Die Flüssigkeitskartusche 90 wird von unten in den Innenraum eingeschoben und dort verdreht, um in Eingriff mit einem in Fig. 2 nur teilweise zu erkennenden Zwischenhebel 70 zu langen. Im eingesetzten Zustand liegt ein Austragstutzen 96 der Flüssigkeitskartusche 90 am unteren Ende des Flüssigkeitsapplikators 80 an, so dass hier austretende Flüssigkeit in den Flüssigkeitsapplikator 80 einströmt. Der Flüssigkeitsaustrag aus der Flüssigkeitskartusche 90 kann dadurch bewirkt werden, dass ein Speicherkörper 92 der Flüssigkeitskartusche 90 gegen den Austragstutzen 96 gedrückt wird und hierdurch eine interne Pumpeinrichtung 98 der Flüssigkeitskartusche 90 betätigt wird. Zur Erzeugung dieser Bewegung ist der Zwischenhebel 70 vorgesehen, der beim einem Verschwenken über Nocken 94 am Speicherkörper 92 diesen anhebt.

Der Flüssigkeitsapplikator 80 selbst ist vorliegend exemplarisch als zylindrischer Applikator zur oralen Verwendung ausgebildet. Er weist eine Austragstruktur 82 auf, die zur Zerstäubung der Flüssigkeit geeignet ist. Vorliegend ist an einem distalen Ende des Flüssigkeitsapplikators 80 eine Düseneinheit 82 in Form einer Düsenplatte 82 mit einer Vielzahl von feinen Düsenöffnungen vorgesehen. Die Flüssigkeit wird hierdurch gedrückt und dabei zerstäubt.

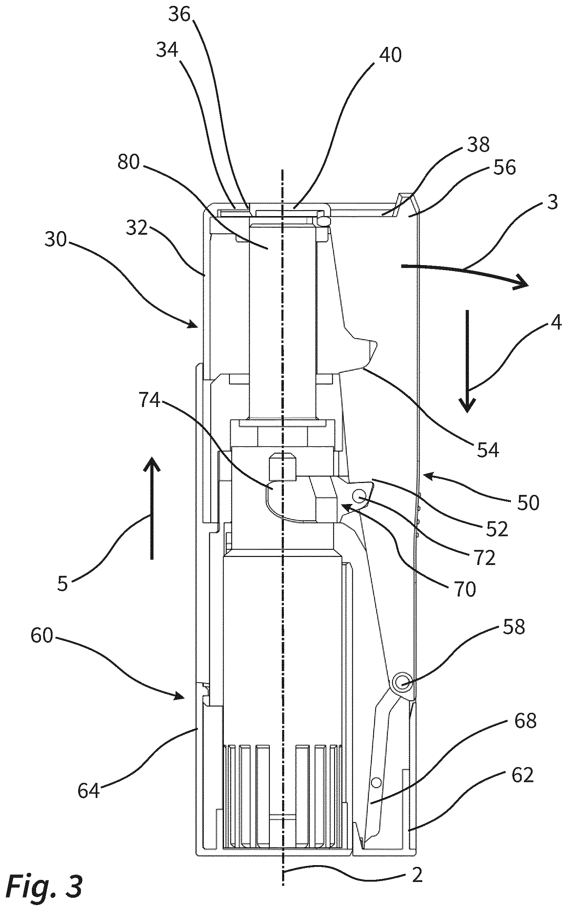

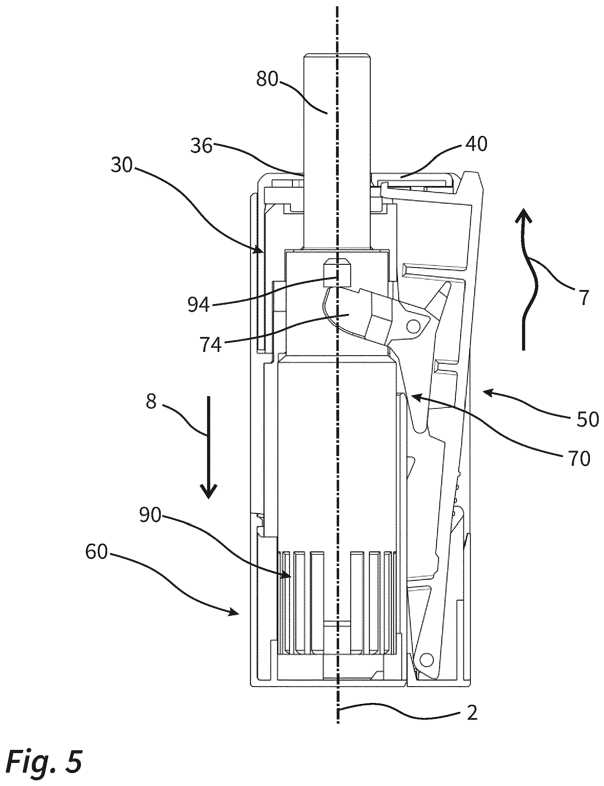

Die Figuren 3 bis 5 verdeutlichen einen Ablauf, mittels dessen der Flüssigkeitsspender 10 aus einem geschützten Zustand in einen nutzbaren Zustand überführt wird, hierbei die Blockiereinrichtungen 4, 72, 40 gelöst werden und abschließend ein Flüssigkeitsaustrag erfolgt.

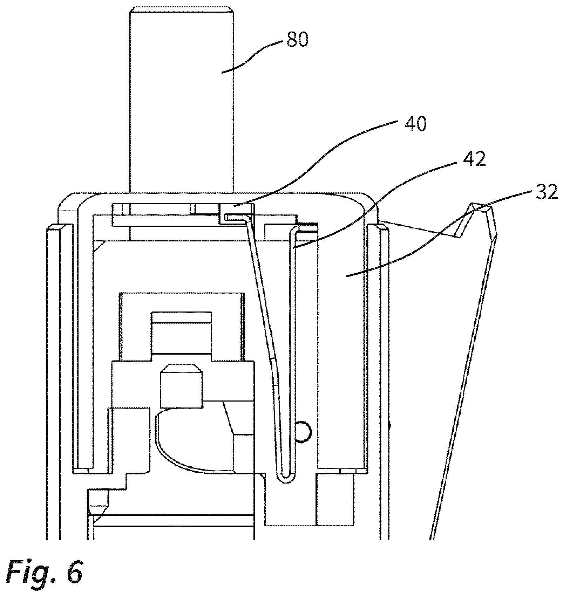

Fig. 3 zeigt den geschützten Zustand. Die Schutzeinheit 30 ist in diesem Zustand in ihrer Schutzstellung, also in ihrer oberen Endlage, so dass sie den Flüssigkeitsapplikator 80 überragt. Der Flüssigkeitsapplikator 80 ist isoliert in einem Innenraum der Schutzeinheit 30 angeordnet. Die Durchbrechung 36 der Schutzeinheit 30 ist in diesem Zustand mittels der bereits genannten Schutzblende 40 verschlossen, die parallel zur Stirnwandung 34 der Schutzeinheit verschiebbar ist und im Zustand der Fig. 1 durch eine in Fig. 6 nochmals verdeutlichte Feder 42 in die Schließstellung gedrückt ist. Der Bedienhebel 50 ist im Zustand der Fig. 3 in angelegter Lage, so dass der Flüssigkeitsspender 10 bezogen auf die Querrichtung eine kompakte Form aufweist.

Eine unmittelbare Verlagerung der Schutzeinheit 30 gegenüber der Applikatoreinheit 60 ist in diesem Zustand nicht möglich, da die genannte Schutzblende 40 eine Blockierung darstellt. Zusätzlich ist eine weitere Blockierung vorgesehen, nämlich dadurch, dass an der Innenseite des Bedienhebels 50 eine erste Aussparung 52 bzw. ein Paar von ersten Aussparungen 52 vorgesehen ist, die sich im Eingriff mit einem Lagerbereich 72 des Zwischenhebels befinden. Eine Oberseite des Lagerbereichs 72 und eine nach unten weisende Seite des Bedienhebels innerhalb der genannten Aussparungen 52 bilden Anschlagsflächen, die beim Versuch der unmittelbaren Verlagerung der Schutzeinheit 30 gegenüber der Applikatoreinheit 60 aneinander anschlagen und eine weitere Verlagerung verhindern.

Um den Flüssigkeitsspender 10 zu verwenden, umgreift der Benutzer das Gehäuse 62 der Applikatoreinheit 60 derart, dass sein Daumen auf dem Bedienhebel 50 aufliegt. Dies stellt die bevorzugte Art des Greifens dar.

Mit dem Daumen kann der Benutzer dann eine Greiferhebung 56 am Bedienhebel 50 ergreifen und diesen in der durch den Pfeil 3 verdeutlichten Art um die Schwenkachse 58 nach außen ausschwenken. Durch diese Bewegung werden beide Blockiereinrichtungen 40, 52, 72 gelöst. Die Aussparung 52 wird so weit nach rechts verlagert, dass sie außer Eingriff mit der Anschlagsfläche des Zwischenhebels 70 kommt. Der Schutzblende 40 wird vom Bedienhebel 50 gegen die Kraft der in Fig. 6 dargestellten Feder 42 nach rechts gezogen und gibt dadurch die Durchbrechung 36 frei.

Ist dieser Zustand erreicht, so kann der Benutzer nun den Bedienhebel 50 zusammen mit der Schutzeinheit 30 gegenüber der Applikatoreinheit nach unten ziehen. In Fig. 3 ist dies durch die Pfeile 4, 5 verdeutlicht. Die gemeinsame Bewegung wird dadurch bewirkt, dass der Bedienhebel50 mit seinem oberen Ende in der bereits genannten Kulisse 38 der Schutzeinheit 30 geführt ist.

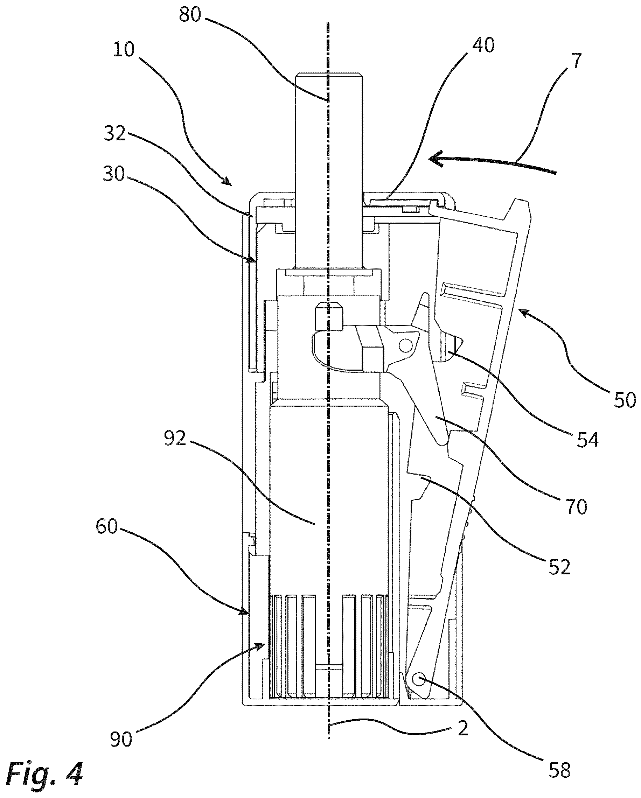

Es stellt sich der Zustand der Fig. 4 ein. Die Schutzeinheit 30 ist nun mit ihrer Schutzhülle 32 in die Applikatoreinheit 60 eingetaucht und der Flüssigkeitsapplikator 80 nimmt eine exponierte Lage ein.

Die Schwenkachse 58 des Bedienhebels 50 hat bei der Bewegung in den Zustand der Fig. 4 ihre Relativlage zur Applikatoreinheit 60 ändern müssen. Dies erfolgt unter Nutzung einer Kulisse 68, die an der Applikatoreinheit 60 vorgesehen ist. Diese Kulisse 68 weist, wie insbesondere aus Fig. 3 zu ersehen ist, einen gegenüber der Längsrichtung 2 schräggestellten Verlauf dar. Zum unteren Ende hin vergrößert sich der Abstand der Kulisse 68 von der Außenwandung der Applikatoreinheit 60. Wirkung dieser Formgebung ist, dass das untere Ende des Bedienhebels 50 bei der Relativverlagerung der Schutzeinheit 30 gegenüber der Applikatoreinheit 60 in das Gehäuse 62 der Applikatoreinheit 60 eintaucht. Es ist daher keine gesonderte Aussparung im Gehäuse 62 erforderlich, durch die im Schutzzustand Verschmutzung eindringen könnte.

Ausgehend vom Zustand der Fig. 4 ist nun, noch immer als Teil des gleichen Bewegungsablaufs des Daumens, die eigentliche Betätigung des Flüssigkeitsspenders 10 möglich, um den Flüssigkeitsaustrag zu erzielen. Hierfür wird der Bedienhebel 50 nun entgegengesetzt zur vorangegangenen Bewegung in Richtung des Pfeils 3 nach innen geschwenkt, wie durch Pfeil 6 verdeutlicht. Diese Schwenkbewegung erfolgt um die Schwenkachse 58 des Bedienhebels, die nun jedoch relativ zur Applikatoreinheit 60 in veränderter Position angeordnet ist.

Eine weitere Aussparung 54 bzw. ein weiteres Paar Aussparungen 54 am Bedienhebel 50 gestatten die Bewegung des Bedienhebels 50 trotz des Zwischenhebels 70 und der dortigen Anschlagsflächen. Die Schutzblende 40, die zuvor gemeinsam mit dem Bedienhebel 50 verlagert wurde, wird bei dieser Bewegung des Bedienhebels 50 nicht mitgeführt, sondern verbleibt in Anlage am Flüssigkeitsapplikator 80. Die Verbindung zwischen dem Bedienhebel 50 und der Schutzblende 80 ist zu diesem Zweck unidirektional ausgebildet. Der Bedienhebel 50 kann die Schutzblende 40 bezogen auf die Fig. 4 zwar nach rechts ziehen, nicht aber nach links drücken.

Durch die Verlagerung des Bedienhebels 50 in Richtung des Pfeils 6 übt dieser ein Moment auf den Zwischenhebel 70 auf, welcher verschwenkt, so dass den Speicherkörper 92 umgreifende Arme 74 von unten gegen die Nocken 94 drücken und dadurch den Speicherkörper 92 als Ganzes anheben. Die innerhalb der Flüssigkeitskartusche 90 vorgesehene Pumpeinrichtung 98 wird hierdurch betätigt und ein Flüssigkeitsaustrag in Form eines feinen Sprühnebels durch die Düseneinheit 82 bewirkt.

Nach erfolgtem Austrag drückt der Benutzer die Applikatoreinheit 60 und den Bedienhebel 50 und damit die Schutzeinheit 30 wieder in Richtung der Ausgangsstellung, wie durch Pfeil 7 und 8 verdeutlicht. Aufgrund der Anschlagsflächen am Zwischenhebel 70 wird der Bedienhebel 50 dabei nochmals leicht nach außen gedrückt, gelangt aber dann wieder in die Ausgangsstellung der Fig. 3. Die Form des Pfeils 7 verdeutlicht den Bewegungsablauf des Bedienhebels 50. Sobald der Flüssigkeitsapplikator 80 durch die Durchbrechung 36 hindurch in den Innenraum der Schutzeinheit 30 gelangt ist, schließt sich die Schutzblende 40 selbsttätig aufgrund der Federkraft der Feder 42.

Der Spender ist nun, nach einer Bewegung, die keinerlei Umgreifen erforderlich gemacht hat und aus dem Schutzzustand in den Nutzzustand, zur Betätigung und zum Flüssigkeitsaustrag sowie abschließend zur Rücküberführung geführt hat, wieder im ursprünglichen Schutzzustand angelangt.

## Patentansprüche

1. Flüssigkeitsspender (10) mit den folgenden Merkmalen:
a. der Flüssigkeitsspender (10) weist einen Flüssigkeitsspeicher (92) und einen Flüssigkeitsapplikator (80) sowie eine Fördereinrichtung (98) zur Förderung von Flüssigkeit aus dem Flüssigkeitsspeicher (92) zum Flüssigkeitsapplikator (80) auf, und
b. der Flüssigkeitsspender (10) verfügt über eine Applikatoreinheit (60) und eine Schutzeinheit (30), und
c. der Flüssigkeitsapplikator (80) ist Teil der Applikatoreinheit (60), und
d. die Schutzeinheit (30) weist eine Schutzhülle (32) zum Schutz der Applikatoreinheit (60) auf, und
e. die Schutzeinheit (30) und die Applikatoreinheit (60) sind zwischen einer Schutzstellung und einer Nutzstellung gegeneinander verlagerbar, und
f. in der Schutzstellung umgibt die Schutzeinheit (30) den Flüssigkeitsapplikator (80) zumindest teilweise und schützt ihn hierdurch, und
g. in der Nutzstellung befindet sich der Flüssigkeitsapplikator (80) zum Zwecke des Flüssigkeitsaustrags relativ zur Schutzeinheit (30) in einer exponierten Position, und
h. der Flüssigkeitsspender (10) weist eine gegenüber der Applikatoreinheit (60) und gegenüber der Schutzeinheit (30) bewegliche Bedienhandhabe (50) auf,
- mittels derer die Schutzeinheit (30) gegenüber der Applikatoreinheit (60) aus der Schutzstellung in die Nutzstellung verlagerbar ist, und
- mittels derer in der Nutzstellung die Fördereinrichtung (98) betätigt werden kann und somit Flüssigkeit aus dem Flüssigkeitsspeicher (92) zum Flüssigkeitsapplikator (80) gefördert werden kann,
**gekennzeichnet durch** das folgende weitere Merkmal:
i. eine Verlagerungsrichtung (3) der Bedienhandhabe (50) ist gegenüber einer Verlagerungsrichtung (2) der Schutzeinheit (30) und der Applikatoreinheit (60) angewinkelt.

2. Flüssigkeitsspender (10) nach Anspruch 1 mit den folgenden weiteren Merkmalen:
a. der Flüssigkeitsspender (10) verfügt über eine Blockiereinrichtung (54, 72, 40), die eine Verlagerung der Schutzeinheit (30) und der Applikatoreinheit (60) aus der Schutzstellung in die Nutzstellung unterbindet, und
b. die Blockiereinrichtung (54, 72, 40) ist mittels der Bedienhandhabe (50) lösbar, so dass anschließend die Verlagerung der Schutzeinheit (30) und der Applikatoreinheit (60) in die Nutzstellung bewirkt werden kann,
vorzugsweise mit einem der folgenden zusätzlichen Merkmale:
c. die Blockiereinrichtung (54, 72, 40) ist derart ausgebildet, dass eine Verlagerung der Schutzeinheit (30) gegenüber der Applikatoreinheit (60) erst beginnen kann, nachdem die Bedienhandhabe (50) aus einer Ausgangsstellung verlagert worden ist, und/oder
d. die Bedienhandhabe (50) ist derart mit der Blockiereinrichtung (54, 72, 40) gekoppelt, dass durch eine Verlagerung der Bedienhandhabe (50) nach außen und insbesondere in eine vom Flüssigkeitsapplikator (80) wegweisenden Richtung die Blockiereinrichtung (54, 72, 40) gelöst werden kann.

3. Flüssigkeitsspender (10) nach Anspruch 1 oder 2 mit dem folgenden weiteren Merkmal:
a. die Bedienhandhabe (50) ist gemeinsam mit der Schutzeinheit (30) gegenüber der Applikatoreinheit (60) verlagerbar,
vorzugsweise mit dem folgenden zusätzlichen Merkmal:
b. es ist eine Kulissenführung (38) vorgesehen, mittels derer die Bedienhandhabe (50) beweglich an der Schutzeinheit (30) geführt ist, wobei die Kulissenführung (38) sich vorzugsweise orthogonal zu einer Verlagerungsrichtung (2) der Schutzeinheit (30) und der Applikatoreinheit (60) erstreckt.

4. Flüssigkeitsspender (10) nach einem der vorstehenden Ansprüche mit dem folgenden weiteren Merkmal:
a. die Bedienhandhabe (50) ist an einer Längsseite des Flüssigkeitsspenders (10) angeordnet und an der hierzu gegenüberliegenden Längsseite ist eine Grifffläche vorgesehen, die Teil der Applikatoreinheit (60) ist,
vorzugsweise mit dem folgenden zusätzlichen Merkmal:
b. die Verlagerungsrichtung (3) der Bedienhandhabe ist gegenüber einer Verlagerungsrichtung (2) der Schutzeinheit (30) und der Applikatoreinheit (60) zumindest phasenweise um einen Winkel von mindestens 75° angewinkelt.

5. Flüssigkeitsspender (10) nach einem der vorstehenden Ansprüche mit dem folgenden weiteren Merkmal:
a. die Bedienhandhabe (50) wird durch einen schwenkbeweglichen Bedienhebel (50) gebildet,
vorzugsweise mit einem der folgenden zusätzlichen Merkmale:
b. der schwenkbewegliche Bedienhebel (50) ist um eine Schwenkachse (58) schwenkbar an der Applikatoreinheit (60) gelagert, die an einem vom Flüssigkeitsapplikator (80) wegweisenden Ende des Bedienhebels (50) vorgesehen ist, und/oder
c. der schwenkbewegliche Bedienhebel (50) kann ausgehend von einer Ruhelage nach außen und/oder von dem Flüssigkeitsapplikator (80) weg verschwenkt werden, um die Blockiereinrichtung (54, 72, 40) zu lösen, und/oder
d. der schwenkbewegliche Bedienhebel (50) kann ausgehend von einer ausgeschwenkten Lage nach innen und/oder in Richtung des Flüssigkeitsapplikators (80) verschwenkt werden, um die Fördereinrichtung (98) zu betätigen.

6. Flüssigkeitsspender (10) nach Anspruch 5 mit dem folgenden weiteren Merkmal:
a. der schwenkbewegliche Bedienhebel (50) ist an der Applikatoreinheit (60) mittels einer Kulissenführung (68) geführt.

7. Flüssigkeitsspender (10) nach Anspruch 6 mit dem folgenden weiteren Merkmal:
a. die Kulissenführung (68) weist eine zumindest abschnittsweise von der Verlagerungsrichtung (2) der Schutzeinheit (30) gegenüber der Applikatoreinheit (60) abweichende Erstreckungsrichtung auf, so dass der Bedienhebel (50) bei der Überführung der Schutzeinheit (30) aus der Schutzstellung in die Nutzstellung im Bereich einer Schwenkachse (58) abweichend von der Verlagerungsrichtung (2) verlagert wird und hierdurch zumindest partiell in ein Gehäuse (62) der Applikatoreinheit (60) eintaucht.

8. Flüssigkeitsspender (10) nach einem der vorstehenden Ansprüche mit dem folgenden weiteren Merkmal:
a. die Blockiereinrichtung (54, 72, 40) umfasst eine Schutzblende (40), die mittels der Bedienhandhabe (50) aus einer Stellung in fluchtender Anordnung zum Flüssigkeitsapplikator (80) in eine demgegenüber versetzten Stellung verlagerbar ist,
vorzugsweise mit dem folgenden zusätzlichen Merkmal:
b. die Schutzeinheit (30) verfügt über eine Stirnwandung (34), in der eine Durchbrechung (36) vorgesehen ist, durch die hindurch der Flüssigkeitsapplikator (80) in der Nutzstellung herausragt und die durch die Schutzblende (40) verschließbar ist

9. Flüssigkeitsspender (10) nach Anspruch 8 mit dem folgenden weiteren Merkmal:
a. die Schutzblende (40) ist mit der Bedienhandhabe (50) unidirektional gekoppelt, so dass durch eine Bewegung der Bedienhandhabe (50) in einer Öffnungsrichtung die Schutzblende (40) in die Öffnungsstellung verlagert werden kann, bei einer Bewegung der Bedienhandhabe (50) in entgegengesetzter Richtung die Schutzblende (40) jedoch in der Öffnungsstellung verbleiben kann.

10. Flüssigkeitsspender (10) nach Anspruch 8 oder 9 mit dem folgenden weiteren Merkmal:
a. der Schutzblende (40) ist ein Federmittel (42) zugeordnet, welches die Schutzblende (40) stets in Richtung der Schließstellung kraftbeaufschlagt.

11. Flüssigkeitsspender (10) nach einem der vorstehenden Ansprüche mit dem folgenden weiteren Merkmal:
a. der Flüssigkeitsspender (10) verfügt über ein Zwischenelement (70), welches in der Nutzstellung des Flüssigkeitsspenders (10) die Betätigung der Bedienhandhabe (50) auf die Fördereinrichtung (98) überträgt,
vorzugsweise mit mindestens einem der folgenden zusätzlichen Merkmale:
b. das Zwischenelement (70) ist als schwenkbeweglicher Zwischenhebel (70) ausgestaltet, mittels dessen die Verlagerung der Bedienhandhabe (50) in einer Betätigungsrichtung in eine Kraftbeaufschlagung der Fördereinrichtung (98) in einer von der Betätigungsrichtung abweichenden Richtung überführt wird, und/oder
c. das Zwischenelement (70) bildet einen Teil der Blockiereinrichtung (54, 72, 40), mittels derer in der Schutzstellung ein Verschieben der Schutzeinheit (30) gegenüber der Applikatoreinheit (60) unterbunden werden kann, und/oder
d. das Zwischenelement (70) und die Bedienhandhabe (50) weisen Anschlagsflächen auf, die in der Schutzstellung das Verschieben der Schutzeinheit gegenüber der Applikatoreinheit unterbinden, wobei vorzugsweise die Anschlagsfläche der Bedienhandhabe (50) in einer ersten Vertiefung (52) der Bedienhandhabe (50) vorgesehen ist und wobei insbesondere vorzugsweise eine zweite Vertiefung (54) an der Bedienhandhabe (50) vorgesehen ist, in die die Anschlagsfläche des Zwischenelements (70) einrückt, wenn mittels der Bedienhandhabe (50) die Fördereinrichtung (98) betätigt wird.

12. Flüssigkeitsspender (10) nach einem der vorstehenden Ansprüche mit dem folgenden weiteren Merkmal:
a. der Flüssigkeitsspender (10) verfügt über eine wechselbare Flüssigkeitskartusche (90),
vorzugsweise mit dem folgenden zusätzlichen Merkmal:
b. der Flüssigkeitsapplikator (80) ist als von einem Außengehäuse (62) der Applikatoreinheit (60) werkzeuglos lösbares Teil vorgesehen, wobei der Flüssigkeitsapplikator (80) vorzugsweise im eingesetzten Zustand in eine Aussparung eingesetzt ist, aus der er werkzeuglos entfernbar ist und/oder auf einen Austragstutzen (96) der Flüssigkeitskartusche (90) derart angepasst ist, dass er gemeinsam mit der Flüssigkeitskartusche (90) entnehmbar ist.

13. Flüssigkeitsspender (10) nach Anspruch 12 mit den folgenden weiteren Merkmalen:
a. die Flüssigkeitskartusche (90) weist einen Speicherkörper (92) zur Bildung des Flüssigkeitsspeicher auf, und
b. die Flüssigkeitskartusche (90) weist einen Austragstutzen (96) auf, und
c. die Fördereinrichtung (98) ist Teil der Flüssigkeitskartusche (90),
vorzugsweise mit dem folgenden weiteren Merkmal:
d. die Fördereinrichtung (98) ist derart ausgestaltet, dass sie durch Relativbewegung des Austragstutzens (96) gegenüber dem Speicherkörper (92) betätigt werden kann, wobei vorzugsweise mittels der Bedienhandhabe (50) der Speicherkörper (92) verlagerbar ist, so dass er gegen den Austragstutzen (96) gedrückt wird und dadurch die Fördereinrichtung (98) betätigt.

14. Flüssigkeitsspender (10) nach einem der vorstehenden Ansprüche mit den folgenden weiteren Merkmalen:
a. die Fördereinrichtung (98) ist als Pumpeinrichtung ausgebildet, oder
b. der Flüssigkeitsspeicher (92) ist als Druckspeicher ausgebildet und die Fördereinrichtung ist als Auslassventil gestaltet, und/oder
c. der Flüssigkeitsapplikator (80) ist zur Herstellung eines zerstäubten Sprühstrahls ausgebildet, wobei hierfür vorzugsweise eine Düseneinheit (82) mit einer Vielzahl von feinen Düsenöffnungen vorgesehen ist, und/oder
d. der Flüssigkeitsspeicher (92) weist ein Volumen von weniger als 50 ml auf, vorzugsweise ein Volumen von weniger als 10 ml, und/oder
e. eine Außenseite der Schutzeinheit (30) und/oder der Applikatoreinheit (60) ist zumindest partiell metallisch oder metallisiert ausgebildet, und/oder
f. die Schutzeinheit (30) und die Applikatoreinheit (60) sind gegeneinander um mindestens 5 mm verschiebbar, vorzugsweise um mindestens 10 mm, insbesondere vorzugsweise um mindestens 15 mm, und/oder
g. der Flüssigkeitsapplikator ist in Form eines Nasalapplikators mit konischer Formgebung ausgebildet, und/oder
h. der Flüssigkeitsapplikator (80) ist als zylindrischer Oralapplikator ausgebildet, und/oder
i. die Schutzeinheit (30), die Applikatoreinheit (60) und die Bedienhandhabe (50) bilden gemeinsam eine geschlossenene Körperform, und/oder
j. auf einer dem Flüssigkeitsapplikator (80) abgewandten Seite ist eine Klappe (64) vorgesehen, die den Austausch der Flüssigkeitskartusche (90) gestattet, und/oder
k. der Flüssigkeitsspender (10) ist Teil eines Sets, welches neben dem Flüssigkeitsspender (10) mitsamt Flüssigkeitskartusche (90) mindestens eine weitere Flüssigkeitskartusche (90) umfasst.
l. der Flüssigkeitsspender (10) weist entlang einer Längsachse eine Länge von maximal 120 mm auf und/oder von einer Länge auf, die mindestens Faktor 2 größer als der maximale Querschnitt ist, und/oder
m. die Schutzeinheit (30) ist innerhalb der Applikatoreinheit (60) geführt,

15. Flüssigkeitsspender (10) nach einem der vorstehenden Ansprüche mit einem der folgenden weiteren Merkmale:
a. der Flüssigkeitsspender (10) ist als Nikotinspender zur oralen Applikation ausgestaltet, oder
b. der Flüssigkeitsspender (10) ist als Nasalspender ausgestaltet, wobei der Flüssigkeitsapplikator durch einen Nasalapplikator gebildet wird.

## Claims

1. Liquid dispenser (10) having the following features:
a. the liquid dispenser (10) has a liquid reservoir (92) and a liquid applicator (80) and also a conveying device (98) for conveying liquid from the liquid reservoir (92) to the liquid applicator (80), and
b. the liquid dispenser (10) has an applicator unit (60) and a protection unit (30), and
c. the liquid applicator (80) is part of the applicator unit (60), and
d. the protection unit (30) has a protective casing (32) for protecting the applicator unit (60), and
e. the protection unit (30) and the applicator unit (60) are displaceable in relation to one another between a protection position and a use position, and,
f. in the protection position, the protection unit (30) at least partially surrounds the liquid applicator (80) and in this way protects it, and,
g. in the use position, the liquid applicator (80) is in an exposed position relative to the protection unit (30) for the purpose of liquid discharge, and
h. the liquid dispenser (10) has an operating handle (50) which is movable in relation to the applicator unit (60) and in relation to the protection unit (30)
- and by means of which the protection unit (30) is displaceable in relation to the applicator unit (60) from the protection position into the use position and
- by means of which, in the use position, the conveying device (98) can be actuated and liquid can thereby be conveyed from the liquid reservoir (92) to the liquid applicator (80),
**characterized by** the following further feature:
i. a displacement direction (3) of the operating handle (50) is angled in relation to a displacement direction (2) of the protection unit (30) and of the applicator unit (60).

2. Liquid dispenser (10) according to Claim 1, having the following further features:
a. the liquid dispenser (10) has a blocking device (54, 72, 40) which prevents displacement of the protection unit (30) and the applicator unit (60) from the protection position into the use position, and
b. the blocking device (54, 72, 40) is releasable by means of the operating handle (50), so that, subsequently, the displacement of the protection unit (30) and the applicator unit (60) into the use position can be brought about,
preferably having one of the following additional features:
c. the blocking device (54, 72, 40) is configured in such a way that displacement of the protection unit (30) in relation to the applicator unit (60) cannot begin until the operating handle (50) has been displaced from a starting position, and/or
d. the operating handle (50) is coupled to the blocking device (54, 72, 40) in such a way that the blocking device (54, 72, 40) can be released by displacement of the operating handle (50) outwards and in particular in a direction which is oriented away from the liquid applicator (80).

3. Liquid dispenser (10) according to Claim 1 or 2, having the following further feature:
a. the operating handle (50) is displaceable together with the protection unit (30) in relation to the applicator unit (60),
preferably having the following additional feature:
b. provision is made of a slotted guide (38) by means of which the operating handle (50) is guided movably on the protection unit (30), wherein the slotted guide (38) preferably extends orthogonally to a displacement direction (2) of the protection unit (30) and of the applicator unit (60).

4. Liquid dispenser (10) according to one of the preceding claims, having the following further feature:
a. the operating handle (50) is arranged on a longitudinal side of the liquid dispenser (10), and provision is made on the opposite longitudinal side of a gripping surface which is part of the applicator unit (60),
preferably having the following additional feature:
b. the displacement direction (3) of the operating handle is, at least in phases, angled at an angle of at least 75° in relation to a displacement direction (2) of the protection unit (30) and of the applicator unit (60).

5. Liquid dispenser (10) according to one of the preceding claims, having the following further feature:
a. the operating handle (50) is formed by a pivotable operating lever (50), preferably having one of the following additional features:
b. the pivotable operating lever (50) is mounted on the applicator unit (60) so as to be pivotable about a pivot axis (58), which is provided at an end of the operating lever (50) that is oriented away from the liquid applicator (80), and/or,
c. proceeding from a rest position, the pivotable operating lever (50) can be pivoted outwards and/or away from the liquid applicator (80) in order to release the blocking device (54, 72, 40), and/or,
d. proceeding from a pivoted-out position, the pivotable operating lever (50) can be pivoted inwards and/or in the direction of the liquid applicator (80) in order to actuate the conveying device (98).

6. Liquid dispenser (10) according to Claim 5, having the following further feature:
a. the pivotable operating lever (50) is guided on the applicator unit (60) by means of a slotted guide (68).

7. Liquid dispenser (10) according to Claim 6, having the following further feature:
a. the slotted guide (68) has a direction of extent which differs at least sectionally from the displacement direction (2) of the protection unit (30) in relation to the applicator unit (60) so that, during the transfer of the protection unit (30) from the protection position into the use position, the operating lever (50) is displaced in the region of a pivot axis (58) differently from the displacement direction (2) and consequently dips at least partially into a housing (62) of the applicator unit (60).

8. Liquid dispenser (10) according to one of the preceding claims, having the following further feature:
a. the blocking device (54, 72, 40) comprises a protective cover (40) which, by means of the operating handle (50), is displaceable from a position in which it is arranged in alignment with the liquid applicator (80) into a position in which it is offset therefrom,
preferably having the following additional feature:
b. the protection unit (30) has an end wall (34) in which provision is made of an aperture (36) through which the liquid applicator (80) protrudes in the use position and which is able to be closed off by the protective cover (40).

9. Liquid dispenser (10) according to Claim 8, having the following further feature:
a. the protective cover (40) is coupled unidirectionally to the operating handle (50) so that, by way of a movement of the operating handle (50) in an opening direction, the protective cover (40) can be displaced into the open position but, if the operating handle (50) is moved in the opposite direction, the protective cover (40) can remain in the open position.

10. Liquid dispenser (10) according to Claim 8 or 9, having the following further feature:
a. the protective cover (40) is assigned a spring means (42) which constantly applies a force in the direction of the closed position to the protective cover (40).

11. Liquid dispenser (10) according to one of the preceding claims, having the following further feature:
a. the liquid dispenser (10) has an intermediate element (70) which, in the use position of the liquid dispenser (10), transmits the actuation of the operating handle (50) to the conveying device (98),
preferably having at least one of the following additional features:
b. the intermediate element (70) is designed as a pivotable intermediate lever (70) by means of which the displacement of the operating handle (50) in an actuating direction is converted into an application of force to the conveying device (98) in a direction different from the actuating direction, and/or
c. the intermediate element (70) forms a part of the blocking device (54, 72, 40) by means of which, in the protection position, a displacement of the protection unit (30) in relation to the applicator unit (60) can be prevented, and/or
d. the intermediate element (70) and the operating handle (50) have abutment surfaces which, in the protection position, prevent the displacement of the protection unit in relation to the applicator unit, wherein preferably the abutment surface of the operating handle (50) is provided in a first depression (52) of the operating handle (50), and wherein in particular preferably a second depression (54) into which the abutment surface of the intermediate element (70) is inserted when the conveying device (98) is actuated by means of the operating handle (50) is provided at the operating handle (50).

12. Liquid dispenser (10) according to one of the preceding claims, having the following further feature:
a. the liquid dispenser (10) has an exchangeable liquid cartridge (90),
preferably having the following additional feature:
b. the liquid applicator (80) is provided as a part which is releasable from an outer housing (62) of the applicator unit (60) without any tools, wherein, in the inserted state, the liquid applicator (80) is preferably inserted into a cutout from which it is removable without any tools and/or is adapted to a discharge connector (96) of the liquid cartridge (90) in such a way as to be removable together with the liquid cartridge (90).

13. Liquid dispenser (10) according to Claim 12, having the following further features:
a. the liquid cartridge (90) has a reservoir body (92) for forming the liquid reservoir, and
b. the liquid cartridge (90) has a discharge connector (96), and
c. the conveying device (98) is part of the liquid cartridge (90),
preferably having the following further feature:
d. the conveying device (98) is configured in such a way as to be actuatable by way of relative movement of the discharge connector (96) in relation to the reservoir body (92), wherein preferably the reservoir body (92) is displaceable by means of the operating handle (50) so as to be pressed against the discharge connector (96) and thus actuates the conveying device (98).

14. Liquid dispenser (10) according to one of the preceding claims, having the following further features:
a. the conveying device (98) is designed as a pump device, or
b. the liquid reservoir (92) is designed as a pressure reservoir and the conveying device is configured as an outlet valve, and/or
c. the liquid applicator (80) is configured to produce an atomized spray jet, wherein, for this purpose, preferably a nozzle unit (82) with a multiplicity of fine nozzle openings is provided, and/or
d. the liquid reservoir (92) has a volume of less than 50 ml, preferably a volume of less than 10 ml, and/or
e. an outer side of the protection unit (30) and/or of the applicator unit (60) is formed to be at least partially metallic or metallized, and/or
f. the protection unit (30) and the applicator unit (60) are slidable by at least 5 mm, preferably by at least 10 mm, in particular preferably by at least 15 mm, in relation to one another, and/or
g. the liquid applicator is designed in the form of a nasal applicator with a conical shaping, and/or
h. the liquid applicator (80) is designed as a cylindrical oral applicator, and/or
i. the protection unit (30), the applicator unit (60) and the operating handle (50) together form a closed body shape, and/or
j. a flap (64) which allows exchange of the liquid cartridge (90) is provided on a side which is directed away from the liquid applicator (80), and/or
k. the liquid dispenser (10) is part of a set which, besides the liquid dispenser (10) with liquid cartridge (90) included, comprises at least one further liquid cartridge (90),
l. the liquid dispenser (10) has along a longitudinal axis a length of at most 120 mm and/or a length that is greater than the maximum cross section by at least a factor of 2, and/or
m. the protection unit (30) is guided within the applicator unit (60).

15. Liquid dispenser (10) according to one of the preceding claims, having one of the following further features:
a. the liquid dispenser (10) is designed as a nicotine dispenser for oral application, or
b. the liquid dispenser (10) is designed as a nasal dispenser, wherein the liquid applicator is formed by a nasal applicator.

## Revendications

1. Distributeur de liquide (10), présentant les caractéristiques suivantes :
a. le distributeur de liquide (10) présente un réservoir à liquide (92) et un applicateur de liquide (80) ainsi qu'un dispositif de transport (98) pour le transport de liquide à partir du réservoir à liquide (92) vers l'applicateur de liquide (80) et
b. le distributeur de liquide (10) dispose d'une unité d'applicateur (60) et d'une unité de protection (30) et
c. l'applicateur de liquide (80) fait partie de l'unité d'applicateur (60) et
d. l'unité de protection (30) présente une enveloppe de protection (32) pour la protection de l'unité d'applicateur (60) et
e. l'unité de protection (30) et l'unité d'applicateur (60) peuvent être déplacées l'une par rapport à l'autre entre une position de protection et une position d'utilisation et
f. dans la position de protection, l'unité de protection (30) entoure au moins partiellement l'applicateur de liquide (80) et le protège de ce fait et
g. dans la position d'utilisation, l'applicateur de liquide (80), en vue de l'évacuation de liquide, se trouve dans une position exposée par rapport à l'unité de protection (30) et
h. le distributeur de liquide (10) présente une poignée de commande (50) mobile par rapport à l'unité d'applicateur (60) et par rapport à l'unité de protection (30),
- au moyen de laquelle l'unité de protection (30) peut être déplacée par rapport à l'unité d'applicateur (60) à partir de la position de protection dans la position d'utilisation et
- au moyen de laquelle, dans la position d'utilisation, le dispositif de transport (98) peut être actionné et du liquide peut donc être transporté à partir du réservoir à liquide (92) vers l'applicateur de liquide (80),
**caractérisé par** la caractéristique supplémentaire suivante :
i. une direction de déplacement (3) de la poignée de commande (50) est inclinée par rapport à une direction de déplacement (2) de l'unité de protection (30) et de l'unité d'applicateur (60).

2. Distributeur de liquide (10) selon la revendication 1, présentant les caractéristiques supplémentaires suivantes :
a. le distributeur de liquide (10) dispose d'un dispositif de blocage (54, 72, 40), qui empêche un déplacement de l'unité de protection (30) et de l'unité d'applicateur (60) à partir de la position de protection dans la position d'utilisation et
b. le dispositif de blocage (54, 72, 40) peut être libéré au moyen de la poignée de commande (50) de telle sorte que le déplacement de l'unité de protection (30) et de l'unité d'applicateur (60) dans la position d'utilisation peut ensuite être provoqué,
de préférence présentant l'une des caractéristiques additionnelles suivantes :
c. le dispositif de blocage (54, 72, 40) est conçu de telle sorte qu'un déplacement de l'unité de protection (30) par rapport à l'unité d'applicateur (60) ne peut démarrer qu'après le déplacement de la poignée de commande (50) hors d'une position de départ et/ou
d. la poignée de commande (50) est accouplée au dispositif de blocage (54, 72, 40) de telle sorte que le dispositif de blocage (54, 72, 40) peut être libéré par un déplacement de la poignée de commande (50) vers l'extérieur et en particulier dans un sens s'écartant de l'applicateur de liquide (80).

3. Distributeur de liquide (10) selon la revendication 1 ou 2, présentant la caractéristique supplémentaire suivante :
a. la poignée de commande (50) peut être déplacée conjointement avec l'unité de protection (30) par rapport à l'unité d'applicateur (60),
de préférence présentant la caractéristique additionnelle suivante :
b. un guidage à coulisse (38) est prévu, au moyen duquel la poignée de commande (50) est guidée de manière mobile sur l'unité de protection (30), le guidage à coulisse (38) s'étendant de préférence de manière orthogonale par rapport à une direction de déplacement (2) de l'unité de protection (30) et de l'unité d'applicateur (60).

4. Distributeur de liquide (10) selon l'une des revendications précédentes, présentant la caractéristique supplémentaire suivante :
a. la poignée de commande (50) est agencée sur un côté longitudinal du distributeur de liquide (10) et une surface de préhension est prévue sur le côté longitudinal opposé à celui-ci, laquelle surface fait partie de l'unité d'applicateur (60),
de préférence présentant la caractéristique additionnelle suivante :
b. la direction de déplacement (3) de la poignée de commande est inclinée au moins par phases d'un angle d'au moins 75° par rapport à une direction de déplacement (2) de l'unité de protection (30) et de l'unité d'applicateur (60).

5. Distributeur de liquide (10) selon l'une des revendications précédentes, présentant la caractéristique supplémentaire suivante :
a. la poignée de commande (50) est formée par un levier de commande (50) pouvant pivoter,
de préférence présentant l'une des caractéristiques additionnelles suivantes :
b. le levier de commande (50) pouvant pivoter est logé de manière à pouvoir pivoter autour d'un axe de pivotement (58) sur l'unité d'applicateur (60), qui est prévu sur une extrémité opposée à l'applicateur de liquide (80) du levier de commande (50) et/ou
c. le levier de commande (50) pouvant pivoter peut être pivoté, partant d'une position de repos, vers l'extérieur et/ou à l'opposé de l'applicateur de liquide (80) afin de libérer le dispositif de blocage (54, 72, 40) et/ou
d. le levier de commande (50) pouvant pivoter peut être pivoté, partant d'une position pivotée vers l'extérieur, vers l'intérieur et/ou en direction de l'applicateur de liquide (80), afin d'actionner le dispositif de transport (98).

6. Distributeur de liquide (10) selon la revendication 5, présentant la caractéristique supplémentaire suivante :
a. le levier de commande (50) pouvant pivoter est guidé sur l'unité d'applicateur (60) au moyen d'un guidage à coulisse (68).

7. Distributeur de liquide (10) selon la revendication 6, présentant la caractéristique supplémentaire suivante :
a. le guidage à coulisse (68) présente une direction d'extension s'écartant au moins par sections de la direction de déplacement (2) de l'unité de protection (30) par rapport à l'unité d'applicateur (60), de telle sorte que le levier de commande (50) est déplacé, lors du passage de l'unité de protection (30) à partir de la position de protection dans la position d'utilisation, dans la zone d'un axe de pivotement (58) en s'écartant de la direction de déplacement (2) et pénètre ainsi au moins partiellement dans un boîtier (62) de l'unité d'applicateur (60).

8. Distributeur de liquide (10) selon l'une des revendications précédentes, présentant la caractéristique supplémentaire suivante :
a. le dispositif de blocage (54, 72, 40) comprend un cache de protection (40), qui peut être déplacé au moyen de la poignée de commande (50) à partir d'une position en alignement avec l'applicateur de liquide (80) dans une position décalée par rapport à celle-ci,
de préférence présentant la caractéristique additionnelle suivante :
b. l'unité de protection (30) dispose d'une paroi frontale (34), dans laquelle est prévue une ouverture (36), à travers laquelle l'applicateur de liquide (80) fait saillie dans la position d'utilisation et qui peut être fermée par le cache de protection (40).

9. Distributeur de liquide (10) selon la revendication 8, présentant la caractéristique supplémentaire suivante :
a. le cache de protection (40) est accouplé de manière unidirectionnelle à la poignée de commande (50) de telle sorte que par un mouvement de la poignée de commande (50) dans un sens d'ouverture, le cache de protection (40) peut être déplacé dans la position d'ouverture et, lors d'un mouvement de la poignée de commande (50) dans le sens opposé, le cache de protection (40) peut néanmoins rester dans la position d'ouverture.

10. Distributeur de liquide (10) selon la revendication 8 ou 9, présentant la caractéristique supplémentaire suivante :
a. un moyen à ressort (42), qui sollicite toujours par une force le cache de protection (40) en direction de la position de fermeture, est associé au cache de protection (40).

11. Distributeur de liquide (10) selon l'une des revendications précédentes, présentant la caractéristique supplémentaire suivante :
a. le distributeur de liquide (10) dispose d'un élément intermédiaire (70) qui transfère, dans la position d'utilisation du distributeur de liquide (10), l'actionnement de la poignée de commande (50) au dispositif de transport (98),
de préférence présentant au moins l'une des caractéristiques additionnelles suivantes :
b. l'élément intermédiaire (70) est conçu comme un levier intermédiaire (70) pouvant pivoter, au moyen duquel le déplacement de la poignée de commande (50) dans un sens d'actionnement est transféré en une sollicitation par la force du dispositif de transport (98) dans un sens s'écartant du sens d'actionnement et/ou
c. l'élément intermédiaire (70) forme une partie du dispositif de blocage (54, 72, 40), au moyen duquel, dans la position de protection, un déplacement de l'unité de protection (30) par rapport à l'unité d'applicateur (60) peut être empêché et/ou
d. l'élément intermédiaire (70) et la poignée de commande (50) présentent des surfaces de butée qui empêchent, dans la position de protection, le déplacement de l'unité de protection par rapport à l'unité d'applicateur, de préférence, la surface de butée de la poignée de commande (50) étant prévue dans un premier renfoncement (52) de la poignée de commande (50) et, en particulier de préférence, un deuxième renfoncement (54) étant prévu sur la poignée de commande (50), dans lequel la surface de butée de l'élément intermédiaire (70) rentre lorsque le dispositif de transport (98) est actionné au moyen de la poignée de commande (50).

12. Distributeur de liquide (10) selon l'une des revendications précédentes, présentant la caractéristique supplémentaire suivante :
a. le distributeur de liquide (10) dispose d'une cartouche de liquide (90) pouvant être remplacée,
de préférence présentant la caractéristique additionnelle suivante :
b. l'applicateur de liquide (80) est prévu comme une pièce pouvant être libérée sans outil du boîtier externe (62) de l'unité d'applicateur (60), l'applicateur de liquide (80) étant de préférence inséré, dans un état d'insertion, dans un évidement dont il peut être enlevé sans outil et/ou étant adapté à une tubulure d'évacuation (96) de la cartouche de liquide (90) de telle sorte qu'il peut être prélevé conjointement avec la cartouche de liquide (90).

13. Distributeur de liquide (10) selon la revendication 12, présentant les caractéristiques supplémentaires suivantes :
a. la cartouche de liquide (90) présente un corps de réservoir (92) pour la formation du réservoir à liquide et
b. la cartouche de liquide (90) présente une tubulure d'évacuation (96) et
c. le dispositif de transport (98) fait partie de la cartouche de liquide (90),
de préférence avec la caractéristique supplémentaire suivante :
d. le dispositif de transport (98) est conçu de telle sorte qu'il peut être actionné par un mouvement relatif de la tubulure d'évacuation (96) par rapport au corps de réservoir (92), de préférence, la poignée de commande (50) permettant de déplacer le corps de réservoir (92) de telle sorte qu'il est comprimé contre la tubulure d'évacuation (96) et actionne ainsi le dispositif de transport (98).

14. Distributeur de liquide (10) selon l'une des revendications précédentes, présentant les caractéristiques supplémentaires suivantes :
a. le dispositif de transport (98) est conçu comme un dispositif de pompe ou
b. le réservoir à liquide (92) est conçu comme un réservoir sous pression et le dispositif de transport est réalisé comme une valve de sortie et/ou
c. l'applicateur de liquide (80) est conçu pour la réalisation d'un jet de pulvérisation atomisé, une unité de buse (82) présentant une multitude de fines ouvertures de buse étant de préférence prévue à cette fin et/ou
d. le réservoir à liquide (92) présente un volume inférieur à 50 ml, de préférence un volume inférieur à 10 ml, et/ou
e. un côté externe de l'unité de protection (30) et/ou de l'unité d'applicateur (60) est conçu au moins partiellement en métal ou de manière métallisée et/ou
f. l'unité de protection (30) et l'unité d'applicateur (60) sont déplaçables en translation l'une par rapport à l'autre d'au moins 5 mm, de préférence d'au moins 10 mm, en particulier de préférence d'au moins 15 mm et/ou
g. l'applicateur de liquide est conçu sous forme d'un applicateur nasal présentant une forme conique et/ou
h. l'applicateur de liquide (80) est conçu comme un applicateur buccal cylindrique et/ou
i. l'unité de protection (30), l'unité d'applicateur (60) et la poignée de commande (50) forment conjointement une forme de corps fermé et/ou
j. un clapet (64), qui permet le remplacement de la cartouche de liquide (90), est prévu sur un côté opposé à l'applicateur de liquide (80) et/ou
k. le distributeur de liquide (10) fait partie d'un ensemble qui comprend, outre le distributeur de liquide (10) avec la cartouche de liquide (90), au moins une autre cartouche de liquide (90) supplémentaire,
l. le distributeur de liquide (10) présente, le long d'un axe longitudinal, une longueur d'au maximum 120 mm et/ou est d'une longueur qui est supérieure d'au moins un facteur 2 à la section transversale maximale et/ou
m. l'unité de protection (30) est guidée à l'intérieur de l'unité d'applicateur (60).

15. Distributeur de liquide (10) selon l'une des revendications précédentes, présentant l'une des caractéristiques supplémentaires suivantes :
a. le distributeur de liquide (10) est conçu comme un distributeur de nicotine pour une application par voie orale ou
b. le distributeur de liquide (10) est conçu comme un distributeur nasal, l'applicateur de liquide étant formé par un applicateur nasal.
